Europäisches Patentamt

European Patent Office

Office européen des brevets

(11) Veröffentlichungsnummer: **0 352 400 B1**

(12) **EUROPÄISCHE PATENTSCHRIFT**

(45) Veröffentlichungstag der Patentschrift: **28.04.93**

(21) Anmeldenummer: **89102248.5**

(22) Anmeldetag: **09.02.89**

(51) Int. Cl.⁵: **A61B 6/00**

(54) **Röntgengerät.**

(30) Priorität: **28.07.88 DE 3825709**

(43) Veröffentlichungstag der Anmeldung:
**31.01.90 Patentblatt 90/05**

(45) Bekanntmachung des Hinweises auf die
Patenterteilung:
**28.04.93 Patentblatt 93/17**

(84) Benannte Vertragsstaaten:
**DE FR**

(56) Entgegenhaltungen:
**EP-A- 0 220 501**
**DE-A- 2 108 657**
**FR-A- 2 194 402**
**US-A- 4 207 595**

(73) Patentinhaber: **SIEMENS AKTIENGESELL-**
**SCHAFT**
**Wittelsbacherplatz 2**
**W-8000 München 2(DE)**

(72) Erfinder: **Horbaschek, Heinz, Dipl.-Ing. (FH)**
**Dreibergstrasse 10a**
**W-8520 Erlangen(DE)**
Erfinder: **Bleitner, Ulrich, Dipl.-Ing. (FH)**
**Dürrmühle 1**
**W-8624 Ebersdorf(DE)**

## Beschreibung

Die Erfindung betrifft ein Röntgengerät zur Erstellung von Röntgenbildern mit einer verstellbaren Röntgenröhre, einer Patientenliege und einem verstellbaren Bildaufnahmesystem als Anlagekomponenten, das einen Bildverstärker und eine daran angekoppelte Fernsehkamera aufweist, mit einer Steuervorrichtung für die Anlagekomponenten und einem Bildwiedergabesystem.

In der DE-A-3 536 079 ist eine Röntgendiagnostikeinrichtung beschrieben, bei der die Anlagekomponenten, die Röntgenröhre, die Patientenliege und das Bildaufnahmesystem, an Roboterarmen angebracht sind, so daß sie sich dreidimensional frei beweglich verstellen lassen. Dadurch tritt aber das Problem auf, daß die Fernsehkamera des Bildaufnahmesystems beliebig ausgerichtet ist, so daß eine Zuordnung der Ausrichtung des Röntgenbildes zu den Gegebenheiten nicht mehr erfolgen kann. Dabei ist es aber für den Arzt wichtig, daß das am Monitor dargestellte Bild beispielsweise in einer Lage wiedergegeben wird, die er als Betrachter auf Grund seiner Position zum Patienten erwartet. Bei den Robotergeräten mit mannigfaltigen Projektionsmöglichkeiten kommt der Korrektur der Gerätedrehung um die Achse des Zentralstrahles somit eine große Bedeutung zu.

Aus der US-A-4,207,595 ist eine Röntgendiagnostikeinrichtung zur Erstellung von Schichtbildern bekannt, bei der der Winkel, den die Röntgenanlage zur Schichtebene einnimmt, erfaßt wird und aufgrund dieses Winkels winkelabhängige Ablenkspannungen berechnet werden, die kissenförmige Verzerrungen der Röntgenbilder aufgrund des Winkels elektronisch ausgleichen. Eine Korrektur der Gerätedrehung um die Achse des Zentralstrahles erfolgt hierbei jedoch nicht.

In der FR-A-2 194 402 ist ein Deckenstativ für eine Röntgendiagnostikeinrichtung beschrieben, deren Winkeleinstellungen relativ zu einem Untersuchungstisch mittels spezieller Detektoren bestimmbar und mittels Servomotoren veränderbar ist. Auch hier ist nicht die Möglichkeit gegeben, das Röntgenbild unabhängig von der Drehung des Systems wiederzugeben.

Die Erfindung geht von der Aufgabe aus, bei einem Röntgengerät der eingangs genannten Art einen elektronischen Ausgleich und eine Korrektur der Gerätedrehung um die Achse des Zentralstrahles zu bewirken, so daß das Röntgenbild eine gewünschte Ausrichtung aufweist.

Die Aufgabe wird erfindungsgemäß dadurch gelöst, daß Mittel zur Erfassung der Lage eines Bezugssystems und mit den Mitteln verbunde Rechenmittel vorgesehen sind, die eine winkelabhängige Überlagerung der Ablenkspannungen ermitteln und mit Ablenkstufen für die Fernsehkamera derart verbunden sind, daß eine Abtastung des Targets der Fernsehkamera unabhängig von der Drehung der Bildaufnahmevorrichtung in Ausrichtung des Bezugssystems erfolgt. Dadurch wird erreicht, daß durch die Mittel die Lage des Bezugssystems erkannt wird. Durch den Rechner erfolgt eine Auswertung dieser Lage und eine Überlagerung der Horizontal- und Vertikalablenkspannungen, so daß die Ablenkung der Fernsehkamera immer derart erfolgt, daß die Abtastrichtung senkrecht auf der gewünschten Lage des Röntgenbildes, beispielsweise auf der Ausrichtung des Bezugssystems, erfolgt.

Bei feststehendem Röntgengerät kann eine entsprechende Korrektur automatisch erfolgen, wenn an wenigstens einer der Anlagekomponenten Positionsgeber angeordnet sind, die den Drehwinkel des Bildaufnahmesystems zu dem Bezugssystem erfassen, und wenn an die Positionsgeber der Rechner angeschlossen ist. Durch die Positionsgeber, die beispielsweise bei starrer Zuordnung des Bezugssystems nur an dem Bildaufnahmesystem angeschlossen sein müssen, ermittelt der Rechner den Drehwinkel des Bildaufnahmesystems zur Ausrichtung des Bezugssystems, beispielsweise der Patientenliege.

Ein einfaches Bezugssystem erhält man, wenn es die Patientenliege ist. Eine Wiedergabe des Röntgenbildes in der Lage, in der die Untersuchungsperson auf Grund ihrer Position zum Patienten es erwartet, kann erreicht werden, wenn das Bezugssystem die Ausrichtung einer Untersuchungsperson zur Röntgenanlage ist, wenn eine Eingabevorrichtung für eine Position der Untersuchungsperson mit dem Rechner verbunden ist und wenn der Rechner derart ausgebildet ist, daß der Winkel zwischen der Position und der Ablenkeinheit der Fernsehkamera in Bezug auf den Zentralstrahl der Röntgenröhre ermittelt wird.

Eine vorteilhafte Ausbildung des Rechners erhält man, wenn er eine erste Stufe aufweist, in der aus den Stellungen der Positionsgeber der Winkel bestimmt wird, wenn in einer daran angeschlossenen zweiten Stufe die diesem Winkel entsprechenden Sinus- und Kosinusfunktionen ermittelt werden, wenn an dieser zweiten Stufe Multiplikationsstufen angeschlossen sind, deren zweite Eingänge mit den jeweiligen Sägezahngeneratoren verbunden sind, und wenn in nachgeschalteten Additionsstufen die Ausgangssignale von jeweils zwei Multiplikationsstufen zusammengefaßt werden, deren Ausgangssignale den Ablenkstufen zugeführt werden, wobei der der Ablenkstufe entsprechende Sägezahn mit der Kosinusfunktion und der der Ablenkstufe nicht entsprechende Sägezahn mit der Sinusfunktion multipliziert wird.

Eine beliebige Einstellung des Röntgenbildes läßt sich auch bei einem verfahrbaren Röntgengerät erreichen, wenn zwischen Fernsehkamera und Bildwiedergabesystem Mittel angeordnet sind, die eine Einblendung von Marken als Bezugssystem in das auf dem Bildwiedergabesystem abgebildete Röntgenbild bewirken, wenn Einstellmittel für die Lage der Marken mit den Mitteln verbunden sind und wenn die Mittel an dem Rechner angeschlossen sind. Eine beliebige, freiwählbare Einstellung kann erfolgen, wenn die Mittel derart ausgebildet sind, daß zwei Marken in das Röntgenbild eingeblendet werden, wobei die erste Marke das Bezugssystem und die zweite Marke die Lage des Bezugssystems kennzeichnet. Die Einstellung vereinfacht sich, wenn die Mittel derart ausgebildet sind, daß eine Marke in das Röntgenbild eingeblendet wird, die die gewünschte Lage kennzeichnet, wobei die Senkrechte auf dem Bildwiedergabesystem das Bezugssystem ist.

Die Erfindung ist nachfolgend anhand eines in der Zeichnung dargestellten Ausführungsbeispieles näher erläutert. Es zeigen:

FIG 1 ein schematisch dargestelltes Röntgengerät mit einem erfindungsgemäßen Rechner,

FIG 2 und 3 die geometrischen Verhältnisse bei der Abtastung,

FIG 4 eine weitere erfindungsgemäße Ausführungsform des Röntgengerates und

FIG 5 bis 8 Beispiele zur wahlweisen Einstellung der Bilddrehung.

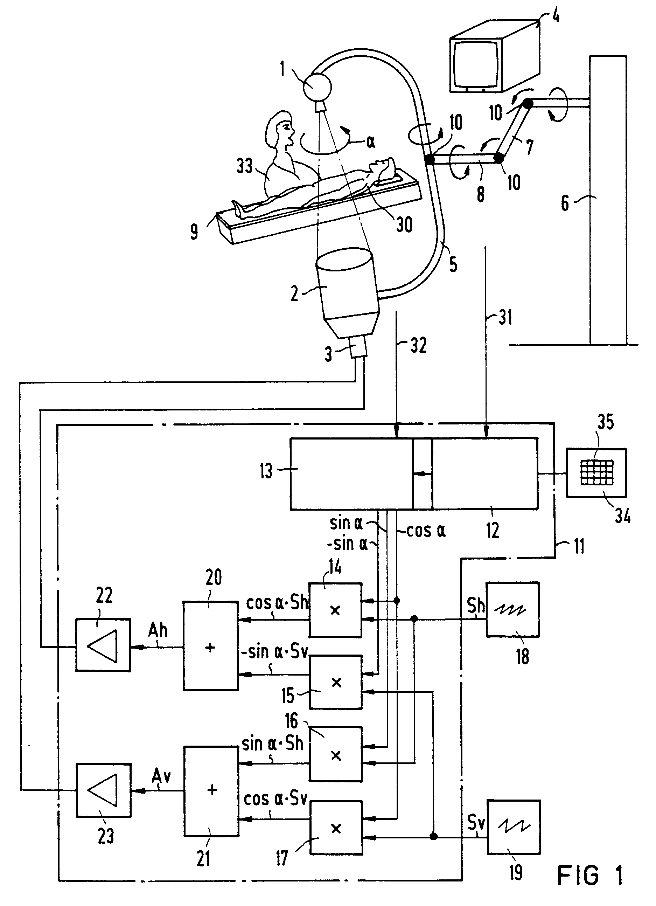

In der FIG 1 ist ein Röntgengerät mit einer Röntgenröhre 1, einem Bildaufnahmesystem mit einem Röntgenbildverstärker 2 und einer Fernsehkamera 3 und als Bildwiedergabesystem einem Monitor 4 dargestellt. Die Röntgenröhre 1 und das Bildaufnahmesystem 2 und 3 sind durch einen sogenannten C-Bogen 5 miteinander verbunden. Sie können aber auch durch Roboterarme getrennt aufgehängt sein. Der C-Bogen 5 ist durch einen Roboterarm mit einer Halterung 6 dreh- und schwenkbar verbunden. Der Roboterarm weist dabei einen an der Halterung 6 dreh- und schwenkbar angebrachten ersten Hebel 7 auf, der an seinem freien Ende mit einem zweiten Hebel 8 schwenkbar verbunden ist, an dem der C-Bogen 5 dreh- und schwenkbar befestigt ist. Zwischen Röntgenröhre 1 und Röntgenbildverstärker 2 ist eine Patientenliege 9 angeordnet, auf der der Patient 30 liegen kann. Der Einfachheit halber sei angenommen, daß die Patientenliege 9 eine feste, nicht veränderliche Ausrichtung zur Halterung 6 aufweist. Dies ist aber nicht unbedingt erforderlich. Sie kann auch, wie bereits erwähnt, von Roboterarmen frei beweglich gehalten werden. In diesem Falle wäre der Drehwinkel der Patientenliege 9 ebenfalls durch Positionsgeber zu ermitteln.

An den Gelenken des Robotorarms sind Positionsgeber 10 angebracht, die über eine erste Leitung 31 mit einem Rechner 11 verbunden sind. Der Rechner 11 weist dabei eine erste Stufe 12 auf, die aus der Stellung der Positionsgeber 10 den Drehwinkel Alpha ermittelt. In einer daran angeschlossenen zweiten Stufe 13 werden aus diesen Werten des Winkels Alpha ($\alpha$) die entsprechenden Sinus- und Kosinus-Funktionen $\sin\alpha$, $-\sin\alpha$ und $\cos\alpha$ gebildet. Diese Funktionen $\sin\alpha$, $-\sin\alpha$ und $\cos\alpha$ werden vier Multiplikationsstufen 14 bis 17 zugeführt, in denen die Ausgangssignale des Generators 18 für die horizontale Sägezahnspannung mit den beiden Funktionen $\sin\alpha$ und $\cos\alpha$ und die Ausgangssignale des Generators 19 für die vertikale Sägezahnspannung mit beiden Funktionen $-\sin\alpha$ und $\cos\alpha$ multipliziert werden. Die Ausgänge von jeweils zwei der Multiplikationsstufen 14 bis 17 werden in Additionsstufen 20 und 21 zusammengefaßt und überlagert. An der Additionsstufe 20 ist die horizontale Ablenkstufe 22 und an die Additionsstufe 21 die vertikale Ablenkstufe 23 angeschlossen. Beide Ablenkstufen 22 und 23 sind mit dem Ablenksatz der Fernsehkamera 3 verbunden.

Der Additionsstufe 20, die ein der horizontalen Ablenkstufe 22 zugeführtes Ausgangssignal Ah erzeugt, wird das Ausgangssignal der ersten Multiplikationsstufe 14 zugeführt, das der Multiplikation der horizontalen Sägezahnspannung Sh mit der Funktion $\cos\alpha$ entspricht. Der zweiten Multiplikationsstufe 15 wird als Ausgangssignal das Produkt der vertikalen Sägezahnspannung Sv mit der Funktion $-\sin\alpha$ entnommen. In der dritten Multiplikationsstufe 16 wird die horizontale Sägezahnspannung Sh mit der Funktion $\sin\alpha$ multipliziert, während die vierte Multiplikationsstufe 17 ein Produkt der vertikalen Sägezahnspannung Sv mit der Kosinusfunktion bildet. Das bedeutet aber, daß den jeweiligen Ablenkstufen 22 und 23 die mit der Kosinus-Funktion multiplizierte entsprechende Sägezahnspannung und die mit der Sinus-Funktion multiplizierte andere Sägezahnspannung zugeführt wird.

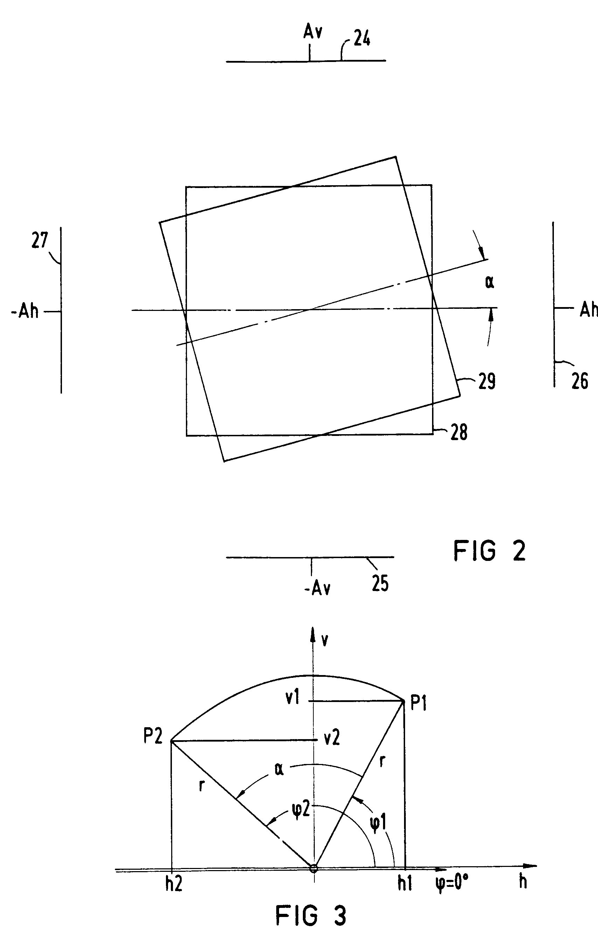

Anhand der FIG 2 werden nun die geometrischen Verhältnisse bei der Abtastung näher erläutert. Das Abtastsystem der Fernsehkamera 3 kann, wie allgemein üblich, magnetisch sein, so daß Ablenkspulen ein entsprechendes Ablenkfeld erzeugen. Die Ablenkung kann aber auch, wie in FIG 2 dargestellt, elektrostatisch erfolgen, wobei an die jeweiligen Ablenkplatten 24 bis 27 die entsprechenden Ablenkspannungen Ah und Av der Ablenkstufen 22 und 23 angelegt werden. Das erste Quadrat 28 gibt dabei die übliche Rasterlage an, wenn an den Ablenkplatten 24 bis 27 die jeweilige Sägezahnspannung S angelegt wird. Soll dagegen eine Ablenkung erfolgen, die gegenüber dem ersten Quadrat 28 um den Winkel Alpha verdreht ist, so muß eine Ablenkung innerhalb des zweiten Quadrates 29 erfolgen. Die nun gebrauchten Ablenkspannungen A müssen eine Ablenkung des Elektronenstrahles der Fernsehkamera 3 bewirken, die senkrecht auf den Seitenlängen dieses zweiten Quadrates 29 liegt. Diese erforderlichen Ablenkspannungen Ah und Av für einen beliebigen Drehwinkel $\alpha$ lassen sich wie folgt mathematisch ermitteln:

Ein beliebiger Punkt P1 mit den rechtwinkligen Koordinaten h1 und v1 und den Winkelkoordinaten r, $\phi$1 soll nach P2 mit den rechtwinkligen Koordinaten h2 und v2 und den Winkelkoordinaten r, $\phi$2 auf einem Kreisbogen mit dem Radius r um den Winkel $\alpha$ gedreht werden (FIG 3).

Es gilt nun:

$$h1 = r \cdot \cos\phi1 \quad (1)$$
$$h2 = r \cdot \cos\phi2 \quad (2)$$
$$v1 = r \cdot \sin\phi1 \quad (3)$$
$$v2 = r \cdot \sin\phi2 \quad (4)$$

Mit $\phi2 = \phi1 + \alpha$ wird:

$$h2 = r \cos (\phi1 + \alpha)$$
$$v2 = r \sin (\phi1 + \alpha)$$

Für die Addition zweier Winkel gilt:

$$\cos = (\phi + \alpha) = \cos\phi \cdot \cos\alpha - \sin\phi \cdot \sin\alpha$$
$$\sin = (\phi + \alpha) = \sin\phi \cdot \cos\alpha + \cos\phi \cdot \sin\alpha$$

und somit:

$$h2 = r \cos\phi1 \cdot \cos\alpha - r \sin\phi1 \cdot \sin\alpha$$
$$v2 = r \sin\phi1 \cdot \cos\alpha + r \cos\phi1 \cdot \sin\alpha$$

Substitution mit (1) und (3) ergibt:

$$h2 = h1 \cos\alpha - v1 \sin\alpha$$
$$v2 = v1 \cos\alpha - h1 \sin\alpha$$

Da der Punkt P1 einen beliebigen Punkt im Quadrat 28 darstellt und gemäß dem Fernsehraster abgelenkt werden darf, ergeben sich die allgemeinen Ablenkfunktionen für die Ablenkspannungen des Quadrates 29:

$$Ah = \cos\alpha \, Sh - \sin\alpha \, Sv$$
$$Av = \cos\alpha \, Sv + \sin\alpha \, Sh.$$

Im bisherigen Falle wurde die Patientenliege 9 als Bezugssystem gewählt, so daß der Winkel Alpha zwischen der Ausrichtung der Patientenliege 9 und der Ausrichtung der vertikalen Ablenkung der Fernsehkamera 3 zu bestimmen war. Dies erfolgte über die Positionsgeber 10. Dem Rechner 11 wurde weiterhin über eine zweite Leitung 32 die Stellung des C-Bogens 5 zugeführt, ob die Anlage im Untertisch- oder Obertischbetrieb arbeitete. Aus diesen Werten ermittelte der Rechner 11 dann die entsprechenden Funktionen.

In manchen Fällen kann es aber vorteilhaft sein, daß nicht die Patientenliege 9 das Bezugssystem ist, sondern die Blickrichtung einer Untersuchungsperson 33 auf den Patienten 30 und so mit auf den Zentralstrahl der Röntgenröhre 1. Hierzu ist am Rechner 11 eine Eingabevorrichtung 34 angeschlossen, über die der ersten Stufe 12 die Position der Untersuchungsperson 33 übermittelt wird. Nach Eingabe der Position beispielsweise als Koordinaten über eine Tastatur 35 ermittelt die erste Stufe 12 des Rechners 11 die Verbindung zwischen der Position und des Zentralstrahles der Rontgenröhre 1 und bestimmt dann auf Grund der Stellungen der Positionsgeber 10 den Winkel Alpha zwischen dieser Verbindung und der jeweiligen Ausrichtung des Röntgengerates.

Durch diese erfindungsgemäße Anordnung erhält man ein Röntgengerät, bei dem eine automatische, elektronische Korrektur der Gerätedrehung erfolgt, so daß das auf dem Monitor 4 wiedergegebene Röntgenbild immer die erforderliche, gewünschte Ausrichtung aufweist.

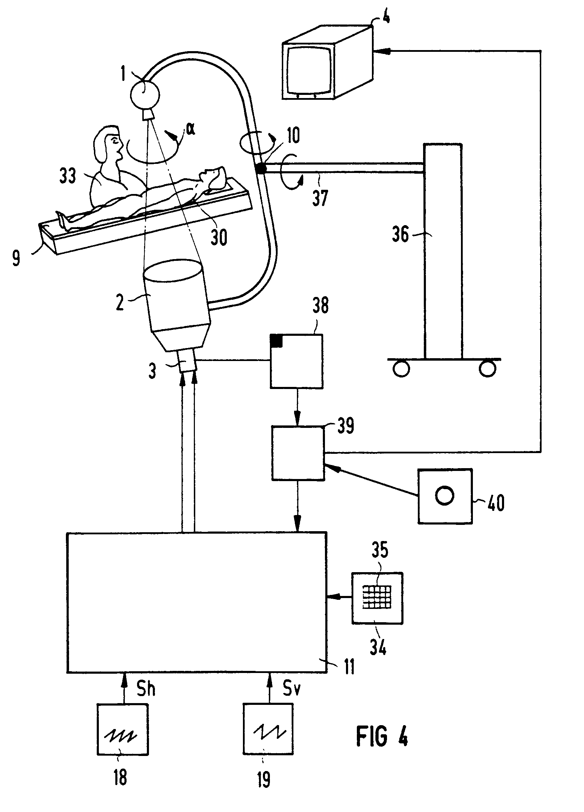

In der FIG 4 ist eine weitere Ausführungsform für ein verfahrbares, bewegliches Röntgengerät 36 dargestellt, das im wesentlichen dem in FIG 1 dargestellten entspricht. Dabei wurden gleiche Teile mit gleichen Bezugszeichen versehen. An dem Röntgengerät 36 ist über einen Tragarm 37 der C-Bogen 5 befestigt, der die Röntgenröhre 1 und den Röntgenbildverstärker 2 mit Fernsehkamera 3 hält. In die in FIG 1 nicht dargestellte Verbindung zwischen der Fernsehkamera 3 und dem Monitor 4 ist ein Bildspeicher 38 für die Röntgenbilder und eine Einblendvorrichtung 39 geschaltet, die, wie noch erklärt wird, Marken in das Fernsehbild einblendet. Diese Marken lassen sich durch Einstellmittel 40 in ihrer Lage verändern. Entweder die Einstellmittel 40 oder, wie dargestellt, die Einblendvorrichtung 39 sind mit dem Rechner 11 verbunden, die die Lage der Marken ihm zuführen, so daß er, wie anhand der FIG 1 beschrieben, daraus die erforderlichen Ablenkspannungen für die Fernsehkamera 3 ermitteln.

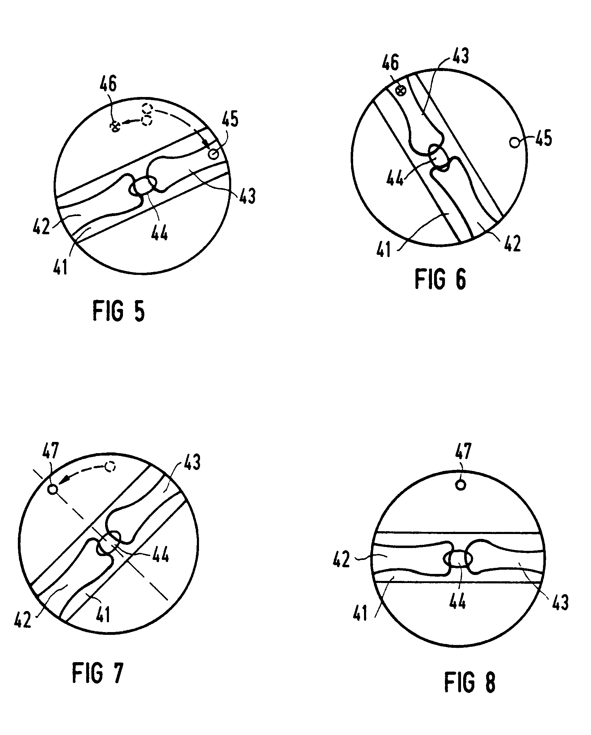

Anhand der FIG 5 bis 8 werden nun Beispiele zu dieser zweiten erfindungsgemäßen Ausführungsform näher erläutert. In allen Figuren sind Röntgenbilder eines menschlichen Beines 41 mit den Beinknochen 43 und 42 und der Kniescheibe 44 dargestellt.

FIG 5 zeigt dabei das Ausgangsbild in der Grundstellung ohne Drehung des Röntgenbildes, wie es beispielsweise nach einer ersten Aufnahme in den Bildspeicher 38 eingelesen wurde. Durch die Einstellmittel 40 wird eine erste Marke 45 auf eine markante Stelle im Röntgenbild, beispielsweise auf das Bein 41, eingestellt, so daß diese erste Marke 45 als Bezugssystem gewertet werden kann. Eine zweite Marke 46 wird nun derart verstellt, daß ihre Lage die gewünschte Lage des Bezugssystemes angibt. Aus dem Winkel, den die beiden Marken 45 und 46 miteinander bilden, ermittelt der Rechner 11 die erforderliche Ablenkung, so daß bei erneutem Einschalten des Röntgengerätes 36 das auf der Bildwiedergabe 4 dargestellte Röntgenbild sich in der gewünschten Lage befindet, wie dies anhand FIG 6 gezeigt ist.

Anstelle von zwei Marken läßt sich nur eine Marke 47 verwenden, wenn als Bezugssystem beispielsweise die senkrechte Ausrichtung im Röntgenbild gewählt wird. Durch die Wahl der Marke 47, die wiederum durch die Einstellmittel 40 in ihrer Lage veränderbar ist, ermittelt der Rechner 11 den Winkel der Marke 47 zu der Bezugsrichtung und daraus die erforderliche Ablenkung, so daß auch hier wieder nach Einschalten der Röntgenanlage das Bild die in FIG 8 dargestellte gewünschte Ausrichtung aufweist.

Durch diese zweite erfindungsgemäße Anordnung erhält man ein Röntgengerät, bei dem eine beliebige elektrodische Korrektur der Gerätedrehung erfolgen kann, so daß auf dem Monitor 4 das Röntgenbild in seiner gewünschten, durch die Einstellmittel 40 beeinflußten Ausrichtung liegt.

**Patentansprüche**

1. Röntgengerät zur Erzeugung von Röntgenbildern mit einer verstellbaren Röntgenröhre (1), einer Patientenliege (9) und einem verstellbaren Bildaufnahmesystem (2, 3) als Anlagekomponenten, das einen Bildverstärker (2) und eine daran angekoppelte Fernsehkamera (3) aufweist, mit einer Steuervorrichtung für die Anlagekomponenten (1 bis 3, 9) und einem Bildwiedergabesystem (4), **dadurch gekennzeichnet,** daß Mittel (10, 45 bis 47) zur Erfassung der Lage eines Bezugssystems und mit den Mitteln (10, 45 bis 47) verbundene Rechenmittel (11) vorgesehen sind, die eine winkelabhängige Überlagerung der Ablenkspannungen ermitteln und mit Ablenkstufen (22, 23) für die Fernsehkamera (3) derart verbunden sind, daß eine Abtastung des Targets der Fernsehkamera (3) unabhängig von der Drehung der Bildaufnahmevorrichtung (2, 3) in Ausrichtung des Bezugssystems erfolgt.

2. Röntgengerät nach Anspruch 1, **dadurch gekennzeichnet,** daß an wenigstens einer Anlagenkomponente (1 bis 3, 9) Positionsgeber (10) angeordnet sind, die den Drehwinkel des Bildaufnahmesystems (2, 3) zu dem Bezugssystem erfassen, und daß an die Positionsgeber (10) der Rechner (11) angeschlossen ist.

3. Röntgengerät nach Anspruch 1 oder 2, **dadurch gekennzeichnet**, daß die Rechenmittel (11) derart ausgebildet sind, daß die Drehung der Bildaufnahmevorrichtung (2, 3) senkrecht zur Ausrichtung des Bezugssystems erfolgt.

4. Röntgengerät nach Anspruch 1, **dadurch gekennzeichnet,** daß das Bezugssystem die Patientenliege (9) ist.

5. Röntgengerät nach Anspruch 1 oder 2, **dadurch gekennzeichnet,** daß eine Eingabevorrichtung (34) für eine Position der Untersuchungsperson mit dem Rechner (11) verbunden ist, daß das Bezugssystem die Ausrichtung einer Untersuchungsperson zur Röntgenanlage ist, und daß der Rechner (11) derart ausgebildet ist, daß der Winkel zwischen der Position und der Anlenkeinheit der Fernsehkamera (3) in Bezug auf den Zentralstrahl der Röntgenröhre (1) ermittelt wird.

6. Röntgengerät nach einem der Ansprüche 1 bis 5, **dadurch gekennzeichnet,** daß der Rechner (11) eine erste Stufe (12) aufweist, in der aus den Stellungen der Positionsgeber (10) der Winkel ($\alpha$) bestimmt wird, daß in einer daran angeschlossenen zweiten Stufe (13) die diesem Winkel ($\alpha$) entsprechenden Sinus- und Kosinus-Funktionen ermittelt werden, daß an dieser zweiten Stufe (13) Multiplikationsstufen (14 bis 17) angeschlossen sind, deren zweite Eingänge jeweils mit Sägezahngeneratoren (18, 19) verbunden sind, und daß in nachgeschalteten Additionsstufen (20, 21) die Ausgangssignale von jeweils zwei Multiplikationsstufen (14 bis 17) zusammengefaßt werden, deren Ausgangssignale den Ablenkstufen (28, 29) zugeführt werden, wobei der der Ablenkstufe (28, 29) entsprechende Sägezahn mit der Kosinus-Funktion und der Ablenkstufe (22, 23) nicht entsprechende Sägezahn mit der Sinus-Funktion multipliziert wird.

7. Röntgengerät nach einem der Ansprüche 1 bis 6, bei der das Röntgengerät beliebig verfahrbar ist, **dadurch gekennzeichnet,** daß zwischen Fernsehkamera (3) und Bildwiedergabesystem (4) Mittel (39) angeordnet sind, die eine Einblendung von Marken (45 bis 47) als Bezugssystem in das auf dem Bildwiedergabesystem (4) abgebildete Röntgenbild bewirken, daß Einstellmittel (40) für die Lage der Marken (45 bis 47) mit den Mitteln (39) verbunden sind, und daß die Mittel (39) an den Rechner (11) angeschlossen sind.

**8.** Röntgengerät nach Anspruch 7, **dadurch gekennzeichnet,** daß die Mittel (39) derart ausgebildet sind, daß zwei Marken (45, 46) in das Röntgenbild eingeblendet werden, wobei die erste Marke (45) das Bezugssystem und die zweite Marke (46) die Lage des Bezugssystems kennzeichnet.

**9.** Röntgengerät nach Anspruch 7, **dadurch gekennzeichnet,** daß die Mittel (39) derart ausgebildet sind, daß eine Marke (47) in das Röntgenbild einblendet wird, die die gewünschte Lage kennzeichnet, wobei die Senkrechte auf dem Bildwiedergabesystem (4) das Bezugssystem ist.

## Claims

**1.** X-ray apparatus for producing X-ray images, having as components of the installation an adjustable X-ray tube (1), a patient support table (9) and an adjustable image pick-up system (2, 3) which has an image intensifier (2) and a television camera (3) coupled thereto, and having a control device for the components of the installation (1 to 3, 9) and an image reproduction system (4), characterised in that there are provided means (10, 45 to 47) for detecting the position of a reference system and calculating means (11) connected to the means (10, 45 to 47), which calculating means (11) determine an angle-dependent superimposition of the deflection voltages and are connected to deflection stages (22, 23) for the television camera (3) in such a way that a scanning of the target of the television camera (3) takes place in alignment with the reference system, independently of the rotation of the image pick-up device (2, 3).

**2.** X-ray apparatus according to claim 1, characterised in that position transmitters (10) are arranged on at least one component of the installation (1 to 3, 9), which position transmitters (10) detect the angle of rotation of the image pick-up system (2, 3) relative to the reference system, and in that the computer (11) is connected to the position transmitters (10).

**3.** X-ray apparatus according to claim 1 or 2, characterised in that the calculating means (11) are formed so that the rotation of the image pick-up device (2, 3) takes place perpendicularly to the alignment of the reference system.

**4.** X-ray device according to claim 1, characterised in that the reference system is the patient support table (9).

**5.** X-ray apparatus according to claim 1 or 2, characterised in that an input device (34) for entering a position of the examining person is connected to the computer (11), in that the reference system is the alignment of an examining person with the X-ray installation, and in that the computer (11) is constructed so that the angle is determined between the position and the link unit of the television camera (3) relative to the central ray of the X-ray tube (1).

**6.** X-ray apparatus according to one of claims 1 to 5, characterised in that the computer (11) has a first stage (12) in which the angle ($\alpha$) is determined from the positions of the position transmitters (10), in that, in a second stage (13) connected thereto, the sine and cosine functions corresponding to this angle ($\alpha$) are ascertained, in that multiplier stages (14 to 17) are connected to this second stage (13), the second inputs of these multiplier stages (14 to 17) being connected respectively to sawtooth voltage generators (18, 19), and in that, in addition stages (20, 21) connected downstream, the output signals from two respective multiplier stages (14 to 17) are combined, the output signals of these stages (14 to 17) being supplied to the deflection stages (28, 29), whereby the sawtooth voltage corresponding to the deflection stage (28, 29) is multiplied by the cosine function and the sawtooth voltage not corresponding to the deflection stage (22, 23) is multiplied by the sine function.

**7.** X-ray apparatus according to one of claims 1 to 6, in which the X-ray apparatus can be moved as desired, characterised in that, between the television camera (3) and the image reproduction system (4), means (39) are arranged which cause a mixing of marks (45 to 47) into the X-ray image displayed on the image reproduction system (4), as a reference system, in that adjusting means (40) for the position of the marks (45 to 47) are connected to the means (39), and in that the means (39) are connected to the computer (11).

**8.** X-ray apparatus according to claim 7, characterised in that the means (39) are constructed in such a way that two marks (45, 46) are mixed into the X-ray image, the first mark (45) identifying the reference system and the second mark (46) identifying the position of the reference system.

**9.** X-ray apparatus according to claim 7, characterised in that the means (39) are constructed in such a way that a mark (47) which identifies the desired position is mixed into the X-ray image, with the perpendicular on the image reproduction system (4) being the reference system.

## Revendications

**1.** Dispositif à rayons X pour générer des images de rayons X comportant, comme éléments constitutifs de l'installation, un tube à rayons X (1) réglable, un plateau de support pour le patient (9) et un système de prise de vues (2,3) déplaçable, qui comporte un amplificateur d'images (2) et une caméra de télévision (3) qui lui est accouplée, et comportant un dispositif de commande pour les éléments constitutifs de l'installation (1 à 3, 9) et un système de restitution des images (4), caractérisé en ce qu'il est prévu des moyens (10,45 à 47) pour détecter la position d'un système de référence et des moyens de calcul (11) qui sont reliés auxdits moyens (10, 45 à 47) et qui détectent une superposition des tensions de déviation en fonction de l'angle et qui sont reliés de telle manière, à l'étage de balayage (22,23) pour la caméra de télévision (3), qu'un balayage de la cible de la caméra de télévision (3) s'effectue indépendamment de la rotation du dispositif de prise de vue (2,3), dans l'alignement du système de référence.

**2.** Dispositif à rayons X selon la revendication 1, caractérisé en ce que des générateurs de position (10) sont associés à au moins un élément constitutif (1 à 3, 9) de l'installation, et détectent l'écart angulaire du système de prise de vues (2,3) par rapport au système de référence, et en ce que l'ordinateur (11) est relié aux générateurs de position (10).

**3.** Dispositif à rayons X selon la revendication 1 ou 2, caractérisé en ce que les moyens de calcul (11) sont réalisés de telle sorte que la rotation du dispositif de prise de vues (2,3) s'effectue perpendiculairement à l'alignement du système de référence.

**4.** Dispositif à rayons X selon la revendication 1, caractérisé en ce que le système de référence est constitué par le plateau de support pour le patient (9).

**5.** Dispositif à rayons X suivant la revendication 1 ou 2, caractérisé en ce qu'un dispositif d'introduction (34) pour une position de la personne à examiner, est relié à l'ordinateur (11), en ce que le système de référence est l'alignement, sur l'installation à rayons X, d'une personne à examiner et en ce que l'ordinateur (11) est réalisé de telle sorte que l'angle entre la position et le dispositif d'articulation de la caméra de télévision (3) est déterminé par rapport au rayon central du tube à rayons X (1).

**6.** Dispositif à rayons X selon l'une des revendications 1 à 5, caractérisé en ce que l'ordinateur (11) comporte un premier étage (12) dans lequel l'angle (α) est déterminé à partir des positions des générateurs de position (10), en ce que les fonctions sinus et cosinus correspondant à cet angle (α) sont déterminées dans un deuxième étage (13) qui est relié au premier étage, en ce que, à ce deuxième étage (13), sont reliés des étages multiplicateurs (14 à 17) dont les deuxièmes entrées sont respectivement reliées aux générateurs d'impulsions en dents de scie (18,19), et en ce que les signaux de sortie de chacun des deux étages de multiplication (14 à 17) sont regroupés dans des étages d'addition (20, 21) montés en aval et dont les signaux de sortie sont envoyés aux étages de balayage (28, 29), la dent de scie correspondant à l'étage de balayage (28, 29) étant multipliée par la fonction cosinus et la dent de scie qui ne correspond pas à l'étage de balayage (22, 23) étant multipliée par la fonction sinus.

**7.** Dispositif à rayons X selon l'une des revendications 1 à 6, dans lequel le dispositif à rayons X est déplaçable de manière quelconque, caractérisé en ce que, entre la caméra de télévision (3) et le système de reproduction d'images (4), sont disposés des moyens (39) qui effectuent une incrustation de repères (45 à 47), comme système de référence, dans l'image à rayons X reproduit sur le système de restitution d'images (4), en ce que les moyens de réglage (40) pour l'état des repères (45 à 47) sont reliés aux moyens (39) et en ce que les moyens (39) sont reliés à l'ordinateur (11).

**8.** Dispositif à rayons X selon la revendication 7, caractérisé en ce que les moyens (39) sont réalisés de telle sorte que deux repères (45, 46) sont incrustés dans l'image à rayons X, le premier repère (45) caractérisant le système de référence et le deuxième repère (46) caractérisant la position du système de référence.

**9.** Dispositif à rayons X selon la revendication 7, caractérisé en ce que les moyens (39) sont réalisés de telle sorte qu'un repère (47) est incrusté dans l'image à rayons X et caractérise l'état souhaité, la verticale du système de restitution de l'image représentant le système de référence.

FIG 1

FIG 2

FIG 3

FIG 4

FIG 5

FIG 6

FIG 7

FIG 8